# EUROPÄISCHE PATENTANMELDUNG

(11) **EP 0 923 926 A2**
(43) Veröffentlichungstag der Anmeldung: **23.06.1999**
(21) Anmeldenummer: 98250427.6
(22) Anmeldetag: 04.12.1998
(51) Int. Cl.: A61K 6/083

(54) **Ionenfreisetzender Kompositwerkstoff**

(30) Priorität: 15.12.1997 DE 19757647
(71) Anmelder: IVOCLAR AG, 9494 Schaan (LI)
(72) Erfinder: Rheinberger, Volker Dr., 9490 Vaduz (LI); Rumphorst, André Dr., 9490 Vaduz (LI); Salz, Ulrich Dr., 88131 Lindau (DE); Grabher, Kurt, 6800 Feldkirch (AT); Fischer, Urs Karl, 9320 Arbon (CH); Moszner, Norbert Prof. Dr., 9492 Eschen (LI)
(74) Vertreter: UEXKÜLL & STOLBERG

(57) **Zusammenfassung**

Die Erfindung betrifft ionenfreisetzende Kompositwerkstoffe auf Basis polymerisierbarer Monomere. Der Werkstoff ist dadurch gekennzeichnet, daß er ein oder mehrere nichtacide, nichtionische, hydrophile Vernetzermonomere, ein oder mehrere nichtacide, nichtionische, hydrophile Verdünnungsmonomere mit einer Viskosität ≤ 1 Pa.s und mindestens einen ionenfreisetzenden Füllstoff enthält. Die Werkstoffe eignen sich insbesondere zur Verwendung als Dentalmaterialien.

## Beschreibung

Die Erfindung betrifft Kompositwerkstoffe auf der Basis eines oder mehrerer nichtacider, nichtionischer, hydrophiler Vernetzermonomere und eines oder mehrerer nichtacider, nichtionischer, hydrophiler Verdünnungsmonomere mit einer Viskosität < 1 Pas, die sich insbesondere als Dentalmaterialien eignen.

Dentalmaterialien, die in der Lage sind, in der Mundhöhle Ionen, wie beispielsweise Fluorid-, Calcium- oder Hydroxid-Ionen freizusetzen, finden aufgrund ihrer remineralisierenden, bioaktiven und kariostatischen Wirkung zunehmend Interesse.

Restaurative Dentalmaterialien, die durch Gehalt an Fluoridquellen, wie speziellen Chlorhexidin-Fluorid-Verbindungen, eine karieshemmende Wirkung entfalten, sind z.B. aus U. Salz, Phillip Journal 14 (1997) 296 bekannt.

Weitere Beispiele für Ionen freisetzende Dentalmaterialien sind Glasionomerzemente und Kompomere, deren organische Matrix zumindest zum Teil aus aciden Monomeren, Oligomeren oder Polymeren aufgebaut ist (A.D. Wilson, J.W. McLean, Glasionomer Cement, Quintessence Publishers, Chicago 1988; J. Nicholson, M. Anstice, Trends Polym. Sci. **2** (1994) 272; R. Hickel, L. Kremers, C. Haffner, Quintessenz **47** (1996) 1581).

Glasionomerzemte sind wasserhaltige, zweikomponentige Zemente auf Basis polymerer organischer Säuren wie beispielsweise Poly(acrylsäure) und pulverförmigen, festen Basen wie Calcium-Fluor-Aluminiumsilikat-Gläsern. Die Aushärtung des Zements erfolgt durch ionische Reaktion zwischen polymergebundenen COOH-Gruppen und den aus dem Füllstoff austretenden Calcium- oder Aluminiumionen, so daß die Komponenten des Glasionomerzements erst kurz vor der Anwendung angemischt werden können. Dies ist umständlich und zudem ist ein Einschluß von Luft meist nicht zu vermeiden, was sich nachteilig auf die Festigkeit des Materials auswirkt. Glasionomerzemente eignen sich aufgrund ihrer schlechten Biegefestigkeit nicht für okklusionstragende Füllungen.

Als Kompomere werden Zusammensetzungen bezeichnet, die aus polymerisierbaren Säuremonomeren und ionenfreisetzenden Glaspartikeln zusammengesetzt sind. Es handelt es sich um wasserfreie Ein-Komponenten-Systeme, die durch radikalische Polymerisation der Monomermatrix aushärten. Eine geringe Säure-Base-Reaktion erfolgt erst dann, wenn über Speichel Wasser in die Füllung aufgenommen wird. Die nicht ausgehärteten Materialien sind feuchtigkeitsempfindlich, und der unkontrollierte Kontakt mit Wasser, beispielsweise bei der Herstellung oder Lagerung, führt zu einer vorzeitigen Aushärtung, die das Material unbrauchbar macht. Kompomere sind belastbarer als Glasionomerzemente, zeigen jedoch häufig eine geringere Ionenfreisetzung.

Sowohl Glasionomerzemente als auch Kompomere weisen im allgemeinen dann ein hohes Ionenfreisetzungsvermögen auf, wenn die Matrix der Materialien einen hinreichenden hydrophilen Charakter besitzt, der eine Wasseraufnahme begünstigt. Im Fall von Glasionomerzementen wird die Matrix durch Polyalkensäuren gebildet, während bei Kompomeren vor allem carbonsäurehaltige Monomere als Matrixmaterialien eingesetzt werden. Da jedoch ein hoher Wassergehalt bzw. eine hohe Wasseraufnahme die mechanischen Eigenschaften von Polymeren nachteilig beeinflußt, war es bisher nicht möglich, Werkstoffe mit einem hohen Ionenfreisetzungsvermögen herzustellen, die gleichzeitig eine große mechanische Belastbarkeit zeigen.

Die EP 0 449 399 B1 offenbart als Unterfüllungsmaterialien geeignete Komposite auf der Basis ionenfreisetzender Füllstoffe und einer Mischung üblicher Dentalmonomere, wie z.B. dem Dimethacrylat von ethoxyliertem Bisphenol-A, einem hydrophoben Dimethacrylat, mit dem Urethandimethacrylat aus 2-Hydroxyethylmethacrylat und 2,2,4-Trimethylhexamethylendiisocyanat, die zwar keine aciden Monomere enthalten, jedoch nur eine geringe Ionenfreisetzung aufweisen.

Der Erfindung liegt die Aufgabe zugrunde, Kompositwerkstoffe mit einem hohen Ionenfreisetzungsvermögen und hoher mechanischer Belastbarkeit bereitzustellen, die im ungehärteten Zustand auch unter feuchten Bedingungen lagerstabil sind und deren mechanische Eigenschaften nach der Aushärtung durch Wasseranlagerung nicht wesentlich beeinträchtigt werden.

Diese Aufgabe wird durch Kompositwerkstoffe auf Basis polymerisierbarer Monomere gelöst, die dadurch gekennzeichnet sind, daß der Werkstoff eine Mischung aus
(a) mindestens einem nichtaciden, nichtionischen, hydrophilen Vernetzermonomer,
(b) mindestens einem nichtaciden, nichtionischen, hydrophilen Verdünnungsmonomer mit einer Viskosität < 1 Pas und
(c) mindestens einen Ionen freisetzenden Füllstoff enthält.

Als Vernetzermonomere werden solche Monomere bezeichnet, die mindestens zwei, vorzugsweise 2 bis 4 polymerisationsfähige Gruppen pro Monomermolekül enthalten.

Die Monomere sind hydrophil, d.h. sie sind zu hydrophilen Wechselwirkungen mit dem Füllstoff in der Lage. Bevorzugt sind Monomere, die eine oder mehrere, vorzugsweise 1 bis 2 Urethan- und/oder OH-Gruppen, vorzugsweise OH-Gruppen enthalten. Es wurde außerdem gefunden, daß diese Gruppen den Ionentransport bzw. die Ionenabgabe fördern.

Unter nichtaciden Verbindungen werden Monomere verstanden, die keine stark aciden Gruppen wie Carboxyl-, Phosphorsäure-, Phoshonsäure-, Phosphinsäure- oder Sulfonsäuregruppen tragen und die vorzugsweise auch keine schwach aciden Gruppen wie phenolische OH-Gruppen oder SH-Gruppen oder CH-acide Gruppen wie β-Diketon- oder β-Diketoestergruppen enthalten.

Nichtionisch im Sinne dieser Erfindung sind Monomere, die keine ionischen Gruppen wie kationische Ammonium- oder Sulfoniumgruppen bzw. anionische Säurerestgruppen der oben genannten stark aciden Gruppen enthalten.

Bevorzugte Vernetzermonomere sind 2,2-Bis-4-(3-methacryloxy-2-hydroxypropyl)-phenylpropan) (Bis-GMA), d.h. das Umsetzungsprodukt von Glycidylmethacrylat und Bisphenol-A (OH-gruppenhaltig), und 7,7,9-Trimethyl-4,13-dioxo-3,14-dioxa-5,12-diazahexadecan-1,16-diyl-dimethacrylat (UDMA), d.h. das Urethandimethacrylat aus 2 mol 2-Hydroxyethylmethacrylat (HEMA) und 1 mol 2,2,4-Trimethylhexamethylendiisocyanat (urethangruppenhaltig). Darüber hinaus sind Umsetzungsprodukte von Glycidylmethacrylat mit anderen Bisphenolen, wie z.B. Bisphenol-B (2,2'-Bis-(4-hydroxyphenyl)-butan), Bisphenol-F (2,2'-Methylendiphenol) oder 4,4'-Dihydroxydiphenyl, sowie Umsetzungsprodukte von 2 mol HEMA oder 2-Hydroxypropyl(meth)acrylat vorzugsweise mit 1 mol bekannter Diisocyanate, wie z.B. Hexamethylendiisocyanat, m-Xylylendiisocyanat oder Toluylendiisocyanat, als Vernetzermonomere bevorzugt.

Als Verdünnungsmonomere werden Monomere mit einer Viskosität von < 1 Pas, vorzugsweise < 100 mPas bezeichnet, die sich zur Verdünnung der im allgemeinen hochviskosen Vernetzermonomere eignen und so die Herstellung von Kompositen mit einem hohen Füllstoffgehalt erlauben. Die Viskositätangaben beziehen sich auf eine Temperatur von 23 °C. Die Bestimmung der Viskosiät erfolgt mittels eines Platten- oder Rotationsviskosimeters gemäß DIN 53018.

Die Verdünnungmonomere enthalten ebenfalls mindestens zwei, vorzugsweise zwei bis drei polymerisationsfähige Gruppen und mindestens eine, vorzugsweise 1 bis 2 OH- und/oder Urethangruppen, vorzugsweise OH-Gruppen. Es handelt sich um nichtionische und nichtacide Verbindungen.

Ein besonders bevorzugtes Verdünnungsmonomer ist Glycerindimethacrylat (GDMA). Andere bevorzugte Verdünnungsmonomere lassen sich durch Umsetzung von niedrigviskosen Di- oder Triepoxiden, wie beispielsweise Ethylenglykoldiglycidylether, Glycerintriglycidylether oder Trimethylolpropantriglycidylether mit (Meth)acrylsäure herstellen. Weiter bevorzugt sind darüber hinaus die Umsetzungsprodukte von 2 bzw. 3 mol Methacrylsäure mit Glycerintriglycidylether oder Trimethylolpropantriglycidylether. Unter "niedrigviskos" werden Substanzen mit einer Viskosität von < 200 mPas, vorzugsweise < 100 mPas verstanden (23 °C).

Bevorzugte polymerisationsfähige Gruppen sind sowohl bei Vernetzungs- als auch bei Verdünnungsmonomeren Methacryl- und/oder Acrylgruppen, insbesondere Methacrylgruppen.

Zur Herstellung von Kompositwerkstoffen werden Vernetzungs- und Verdünnungsmomonere mit Füllstoffen, Initiatoren für die radikalische Polymerisation und ggf. weiteren Hilfstoffen gemischt. Einkomponentige Kompositwerkstoffe, d.h. Kompositwerkstoffe, die alle erforderichen Komponenten enthalten, sind bevorzugt.

Die erfindungsgemäßen Kompositwerkstoffe weisen vorzugsweise folgende Zusammensetzung auf:
(a) 1 bis 40 Gew.-%, besonders bevorzugt 10 bis 30 Gew.-% und ganz besonders bevorzugt 15 bis 25 Gew.-% Vernetzermonomer,
(b) 2 bis 40 Gew.-%, besonders bevorzugt 2 bis 30 Gew.-% und ganz besonders bevorzugt 5 bis 20 Gew.-% Verdünnungsmonomer;
(c) 30,0 bis 94,0 Gew.-% Füllstoff;
(d) 0,01 bis 5 Gew.-%, besonders bevorzugt 0,1 bis 2,0 Gew.-% eines Initiators für die radikalische Polymerisation sowie ggf. weitere Hilfsstoffe.

Der Füllstoffgehalt hängt maßgeblich vom Verwendungszweck des Kompositwerkstoffs ab und beträgt im Fall von Befestigungszementen vorzugsweise 30 bis 60 Gew.-%, besonders bevorzugt 40 bis 60 Gew.-% und im Fall von Füllungskompositen 60 bis 94 Gew.- %, vorzugsweise 70 bis 85 Gew.-%.

Die Kompositwerkstoffe enthalten vorzugsweise mindestens 5 Gew.- %, besonders bevorzugt mindestens 10 Gew.-% hydroxylgruppenhaltige Monomere, d.h. Monomere mit mindestens einer Hydroxylgruppe pro Monomermolekül.

Die ungehärteten Werkstoffe können bis zu 1,0 Gew.-% Wasser enthalten, ohne daß die Lagerbeständigkeit der Materialien oder die mechanischen Eigenschaften der ausgehärteten Werkstoffe beeinträchtigt würden. Dies erleichtert sowohl die Herstellung als auch die Verarbeitung der Materialien durch den Zahnarzt bzw. Zahntechniker erheblich.

Die erfindungsgemäßen Werkstoffe enthalten vorzugsweise maximal 2 Gew.-% an monofunktionellen Monomeren, d.h. Monomeren mit nur einer ungesättigten, polymerisationsfähigen Gruppe, wie beispielsweise 2-Hydroxyethyl(meth)acrylat.

Als Initiatoren für die radikalische Polymerisation eignen sich die bekannten Initiatoren für die Kalt-, Heiß- und Photohärtung. Geeignete Initiatoren werden beispielsweise in der Encyclopedia of Polymer Science and Engineering, Vol. 13, Wiley-Intersci. Pub., New York etc. 1988, S. 754 ff. beschrieben.

Bevorzugte Initiatoren sind Azoverbindungen, wie Azobis(isobutyronitril) (AIBN) oder Azobis(4-cyanvaleriansäure) oder Peroxide, wie Dibenzoylperoxid, Dilauroylperoxid, tert.-Butylperoctoat, tert.-Butylperbenzoat oder Di-(tert.-butyl)-peroxid.

Als Initiatoren für die Heißhärtung eignen sich besonders Benzpinakol und 2,2'-Di(C₁-C₈-alkyl)benzpinakole.

Geeignete Photoinitiatoren für den UV- oder sichtbaren Bereich werden von J.P. Fouassier, J.F. Rabek (Hrsg.), Radiation Curing in Polymer Science and Technology, Vol. II, Elsevier Applied Science, London und New York 1993, Seiten 155 bis 237, beschrieben. Bevorzugte Photoinitiatoren sind Benzoinether, Dialkylbenzilketale, Dialkoxyacetophenone, Acylphosphinoxide, α-Diketone, wie 10-Phenanthrenchinon, Diacetyl, Furil, Anisil, 4,4'-Dichlorbenzil und 4,4'-Dialkoxybenzil und Kampferchinon.

Zur Herstellung von Dentalmaterialien sind Dibenzoylperoxid, Kampferchinon und Acylphosphinoxide bevorzugt.

Als Füllstoffe eignen sich alle zur Herstellung von Glasionomerzementen bekannten ionenfreisetzenden Füllstoffe. Bevorzugt sind Ca²⁺-, F⁻- und/oder OH⁻-ionenfreisetzende Füllstoffe, wie sie in den oben genannten Schriften oder in der DE 39 41 629 und der US-A-4,814,362 beschrieben werden.

Besonders bevorzugte Füllstoffe sind Glaspulver von Fluoraluminiumsilikatgläsern mit einer mittleren Partikelgröße von 0,05 bis 15 µm, vorzugsweise 0,5 bis 5,0 µm, die als Hauptbestandteile Siliciumoxid, Aluminiumoxid und Calciumoxid enthalten (vgl. A.D. Wilson, J.W. McLean, Glasionomerzement, Quintessence Verlags GmbH, Berlin 1988, Seiten 21 ff.).

Bevorzugte Gläser werden durch Aufschmelzen von 25 bis 45 Gew.-% SiO₂, 15 bis 40 Gew.-% Al₂O₃, 0 bis 10 Gew.-% AlF₃, 0 bis 30 Gew.-% CaO, 0 bis 10 Gew.-% Na₂O, 0 bis 15 Gew.-% CaF₂, 0 bis 15 Gew.-% NaF und 0 bis 25 Gew.-% AlPO₄ erhalten.

Ein besonders bevorzugtes Glas weist die folgende Zusammensetzung auf: 25 Gew.-% SiO₂, 16,2 Gew.-% Al₂O₃, 8,8 Gew.-% AlF₃, 12,8 Gew.-% NaF, 13,0 Gew.-% CaF₂ und 24,2 Gew.-% AlPO₄.

In der Zahnheilkunde haben sich Dentalmaterialien bewährt, die Calciumhydroxid oder Fluorid freisetzen. Durch die kontrollierte Abgabe von Calciumhydroxid und Fluorid wird die Bildung von sekundärem Dentin gefördert und eine alkalisierende Wirkung gegenüber der Pulpa erzielt, was diese gegen Säuren und bakterielle Angriffe schützt.

Alkalische Ionen freisetzende Füllstoffe sind jedoch mit aciden Monomeren nicht kompatibel und bewirken eine spontane Härtung der Matrix. Einkomponentige, Calciumhydroxid freisetzende Komposite sind daher entweder nicht stabil oder zeigen bei Verwendung nicht-acider Monomere nur eine geringe Ionenfreisetzung.

Die erfindungsgemäß eingesetzten Monomere enthalten keine aciden Gruppen und können daher problemlos mit alkalischen Füllstoffen kombiniert werden. Sie erlauben erstmals die Herstellung von einkomponentigen, Calciumhydroxid freisetzenden Kompositen mit einem hohen Ionenfreisetzungsvermögen.

Unter alkalischen Füllstoffen werden Füllstoffe mit alkalischen Komponenten wie CaO, Ca(OH)₂ oder Na₂O verstanden, die in Verbindung mit Wasser eine alkalische Reaktion zeigen.

Bevorzugte alkalische Füllstoffe sind Calciumhydroxid, Calciumoxid und insbesondere Calciumhydroxyd freisetzende Gläser, d.h. Gläsern mit einem hohen Calciumoxidgehalt.

Bevorzugt sind Gläser mit einem CaO-Gehalt von mindestens 20 Gew.-%, vorzugsweise 40 bis 75 Gew.-% und insbesondere 45 bis 60 Gew.-%.

Ein bevorzugtes Glaspulver mit hohem Calciumoxidgehalt wird in der EP 0 449 399 B1 beschrieben und enthält 40 bis 75 Gew.-%, vorzugsweise 45 bis 60 Gew.-% Calciumoxid, 5 bis 30 Gew.-%, vorzugweise 15 bis 28 Gew.-% Boroxid und 5 bis 35 Gew.-%, vorzugsweise 10 bis 30 Gew.-% Siliciumdioxid. Die durchschnittliche Teilchengröße (Gewichtsmittel) des Glaspulvers liegt zwischen 1 und 100 µm, vorzugsweise 10 und 30 µm.

Weiter bevorzugte sind transparente Gläser mit hoher Calcium- und Fluorionenabgabe, welche die folgenden Komponenten enthalten:

| Komponente | Gew.-% |
|---|---|
| SiO₂ | 24,0 bis 56,0 |
| CaO | 26,0 bis 57,0 |
| F | 4,0 bis 14,0. |

Die erfindungsgemäß eingesetzten transparenten Gläser enthalten bevorzugt zusätzlich mindestens eine der folgenden Komponenten

| Komponente | Gew.-% |
|---|---|
| | |
| Na₂O | 1,0 bis 9,0 |
| B₂O₃ | 1,0 bis 14,0 |
| MgO | 1,0 bis 14,0 |
| SrO | 1,0 bis 12,0 |
| ZnO | 1,0 bis 7,0 |
| Al₂O₃ | 0,5 bis 5,0 |
| ZrO₂ | 0,5 bis 4,0. |

Für die einzelnen Komponenten der transparenten Gläser existieren bevorzugte Mengenbereiche. Diese können unabhängig voneinander gewählt werden und sind wie folgt

| Komponente | Gew.-% |
|---|---|
| | |
| SiO₂ | 30,0 bis 54,0 insbesondere 36,0 bis 54,0 |
| CaO | 32,0 bis 50,0 |
| F | 5,0 bis 12,0 |
| Na₂O | 1,0 bis 8,0 |
| B₂O₃ | 1,0 bis 12,0 |
| MgO | 1,0 bis 10,0 |
| SrO | 1,0 bis 10,0 |
| ZnO | 1,0 bis 5,0 |
| Al₂O₃ | 0,5 bis 4,0 |
| ZrO₂ | 0,5 bis 4,0. |

Besonders bevorzugte Mengenbereiche der Komponenten des transparenten Glases, die unabhängig voneinander gewählt werden können, sind wie folgt

| Komponente | Gew.-% |
|---|---|
| | |
| SiO₂ | 45,0 bis 54,0 |
| CaO | 35,0 bis 50,0 |
| F | 6,0 bis 12,0 |
| Na₂O | 4,0 bis 7,0 |
| B₂O₃ | 1,0 bis 12,0 |
| MgO | 1,0 bis 10,0 |
| SrO | 1,0 bis 10,0 |
| ZnO | 1,0 bis 5,0 |
| Al₂O₃ | 0,5 bis 4,0 |
| ZrO₂ | 0,5 bis 4,0. |

Alle vorstehend sowie im folgenden in der Beschreibung und den Ansprüchen angegebenen Mengen der Komponenten der transparenten fluorhaltigen Gläser sind als Werte zu verstehen, die wie folgt erhalten wurden. Die Mengen der Oxide wurden durch quantitative Bestimmung der entsprechenden Kationen, d.h. Si, Ca, Na, B, Mg, Sr, Zn nd Al, mittels Röntgenfluoreszenz-Analyse und Umrechnung der erhaltenen Werte in die Mengen an entsprechenden Oxiden ermittelt. Somit wird von dem Gehalt eines Kations auf den Gehalt der entsprechenden Oxide geschlossen. Demgegenüber wird die Menge an F⁻ direkt mittels einer für Fluoridionen selektiven Elektrode bestimmt, nachdem das Glas einem Soda-Pottasche-Aufschluß unterzogen worden war.

Infolge der hohen F-Gehalte der transparenten Gläser kommt es in merklichem Ausmaß zur Bildung von Fluoriden, wie z.B. CaF₂, in dem Glas. Daher sind die errechneten Oxidgehalte und demnach der Absolutgehalt des Glases an Sauerstoff zu hoch, und die Summe der Komponenten überschreitet 100 %. Der über 100 % hinausgehende Anteil wird daher als sogenannter "fluoräquivalenter Sauerstoff" ausgewiesen. Dies ist für fluoridhaltige silicatische Gläser üblich und wird z.B. in J. Lange "Rohstoffe der Glasindustrie", Deutscher Verlag für Grundstoffindustrie, Leipzig, Stuttgart (1993), S. 221-223, ausführlich beschrieben.

Es ist in der Glasherstellung allgemein üblich, Fluoride in geringen Mengen als Flußmittel zuzusetzen, um das Schmelzverhalten des jeweiligen Glases zu verbessern. Durch diese geringen Anteile an Fluor wird jedoch die Gesamtstruktur der Gläser nicht wesentlich verändert.

Demgegenüber ist in dem erfindungsgemäß eingesetzten transparenten Glas ein hoher Fluoranteil von mindestens 4,0 Gew.% eingebaut, der die Grundstruktur des Glases wesentlich gegenüber entsprechenden Gläsern verändert, die frei von Fluor sind oder nur geringe Fluorgehalte infolge eingesetzten Flußmittels haben. Durch diesen hohen Anteil an Fluor und dem gleichzeitigen Einbau von weiteren Netzwerkwandlerionen, wie z.B. Ca²⁺ oder Na⁺ erfolgt ein starker Abbau der SiO₄-Tetraeder-Netzwerkstruktur des Glases. Es entsteht eine Glas struktur, die mit der klassischen Netzwerktheorie nicht mehr zu erklären ist. Die Glasstruktur nähert sich einer neuen Glasstruktur an, die als "Invertglasstruktur" bezeichnet wird. Unter einem Invertglas versteht man ein Glas, das weniger als 50 Mol.-% Netzwerkbildneranteil hat.

Infolge der geänderten Struktur verändert sich vor allem der Brechungsindex des Glases, und es ist überraschenderweise auch eine Fluorionenabgabe bei gleichzeitiger Calciumionenabgabe aus dem Glas möglich. Bei Einsatz des Kompositmaterials im Dentalbereich kann somit in der Mundhöhle durch die Calciumionen zusammen mit Carbonat im Speichel die gewünschte alkalische Wirkung und durch die Fluorionen deren bekannte remineralisierende Wirkung hervorgerufen werden. Auch Calciumionen fördern dem Remineralisierungsprozeß.

Weiter bewirkt der hohe Fluoridanteil des Glases eine starke Absenkung von dessen Brechungsindex auf Werte von unter 1,60 und bevorzugt unter 1,56. Die durch Härtung des polymerisierbaren Monomers entstehende organische Matrix des Kompositmaterials hat einen sehr ähnlichen Brechungsindex, weshalb das gesamte Kompositmaterial ebenfalls transluzent oder sogar transparent sein kann. Dies ist naturgemäß von besonderem Vorteil, wenn das Kompositmaterial für die Herstellung von sichtbaren Dentalrestaurationen eingesetzt werden sollen, die naturgemäß ähnliche optische Eigenschaften wie transluzentes natürliches Dentalmaterial haben sollen.

Zur Herstellung des erfindungsgemäß eingesetzten transparenten Glases werden entsprechende Rohstoffe, insbesondere Oxide, Carbonate und Fluoride, gemischt und bei Temperaturen von insbesondere 1000 bis 1600 °C zu einem Glas aufgeschmolzen. Die entstehende Glasschmelze wird dann durch Eingießen in Wasser abgeschreckt. Die erhaltene transparente Glasfritte wird anschließend gemahlen, getrocknet und kann dann mit polymerisierbarem Monomer zu dem erfindungsgemäßen polymerisierbaren Kompositmaterial kombiniert werden.

Üblicherweise wird das Glas als Pulver eingesetzt, wobei die mittlere Größe der Teilchen üblicherweise 1 bis 100µm und bevorzugt 10 bis 30 µm auf die Teilchenanzahl beträgt.

Die oben genannten ionenfreisetzenden Füllstoffe können mit anderen Füllstoffen kombiniert werden, wobei der Anteil der ionenfreisetzenden Füllstoffe mindestens 5 Gew.-%, vorzugsweise 15 bis 70 Gew.-% beträgt.

Als weitere Füllstoffkomponenten eignen sich insbesondere amorphe, kugelförmige Materialien auf der Basis von Mischoxiden aus SiO₂, ZrO₂ und/oder TiO₂ mit einer mittleren durchschnittlichen Partikelgröße von 0,005 bis 2,0 µm, vorzugsweise von 0,1 bis 1 µm, wie sie beispielsweise in der DE-PS 32 47 800 offenbart werden, mikrofeine Füllstoffe, wie pyrogene Kieselsäure oder Fällungskieselsäure sowie Makro- oder Mini-Füllstoffe, wie Quarz-, Glaskeramik- oder Glaspulver mit einer durchschnittlichen Teilchengröße von 0,01 bis 20 µm, vorzugsweise 0,5 bis 5 µm, sowie röntgenopake Füllstoffe, wie Ytterbiumfluorid. Unter Mini-Füllstoffen werden Füllstoffe mit einer Partikelgröße von 0,5 bis 1,5 µm und unter Makro-Füllstoffen Füllstoffe mit einer Partikelgröße von 10 bis 20 µm verstanden.

Darüber hinaus können die erfindungsgemäßen Zusammensetzungen im Bedarfsfall weitere Hilfsstoffe enthalten, insbesondere Stabilisatoren, UV-Absorber, Farbstoffe, Pigmente und/oder Gleitmittel. Unter Stabilisatoren werden solche Stoffe verstanden, die eine vorzeitige Polymerisation verhindern und damit vor allem die Lagerstabilität von Monomermischungen und Kompositen erhöhen, ohne jedoch die Eigenschaften der ausgehärteten Materialien zu beeinträchtigen. Bevorzugte Stabilisatoren sind Hydrochinonmonomethylether (MEHQ) und 2,6-Di-tert.-butyl-4-methylphenol (BHT).

Es wurde überraschenderweise gefunden, daß durch die gleichzeitige Verwendung der oben genannte Vernetzer- und Verdünnungsmonomere Zusammensetzungen mit einem hohen Ionenfreisetzungvermögen erhalten werden können, die im ungehärteten Zustand auch unter feuchten Bedingungen lagerstabil sind und deren mechanische Eigenschaften durch Wasseranlagerung nicht signifikant verschlechtert werden. Die erfindungsgemäßen Monomermischungen lassen sich problemlos mit alkalischen Füllstoffen zu einkomponentigen Kompositen verarbeiten.

Im folgenden wird die Erfindung anhand von Ausführungsbeispielen weiter erläutert.

### Beispiele 1 bis 5

Als Ausgangsmaterialien für die Herstellung hydrophiler Komposite wurden Monomermischungen mit den in Tabelle 1 angegebenen Zusammensetzungen hergestellt und anschließend zu den in Tabelle 2 gezeigten einkomponentigen Kompositpasten weiterverarbeitet.

**Tabelle 1**

| **Zusammensetzung der Monomermischungen** | | | | | |
|---|---|---|---|---|---|
| Monomer | Mischung (in Masse.%) | | | | |
| | 1 | 2^{*)} | 3 | 4^{*)} | 5^{*)} |
| Bis-GMA¹⁾ | 39,0 | 42,0 | 42,0 | 42,0 | 42,0 |
| UDMA²⁾ | 30,0 | 37,1 | 27,8 | 27,8 | 27,8 |
| GDMA³⁾ | 30,0 | - | 29,4 | - | - |
| TEGDMA⁴⁾ | - | 20,1 | - | 29,4 | - |
| HEMA⁵⁾ | - | - | - | - | 29,4 |
| Initiator/ Additive⁶⁾ | 1,0 | 0,8 | 0,8 | 0,8 | 0,8 |

| | | | | | |
|---|---|---|---|---|---|
| ^{*)} Vergleichsbeispiel | | | | | |
| ¹⁾ Bisphenol-A-glycidylmethacrylat (Fa. Essehem) | | | | | |
| ²⁾ 7,7,9-Trimethyl-4,13-dioxo-3,14-dioxa-5,12-diazahexadecan-1,16-diyl-dimethacrylat (Fa. Ivoclar) | | | | | |
| ³⁾ Glycerindimethacrylat (Fa. Röhm) | | | | | |
| ⁴⁾ Triethylenglykoldimethacrylat (Fa. Esschem) | | | | | |
| ⁵⁾ 2-Hydroxyethylmethacrylat (Fa. Röhm) | | | | | |
| ⁶⁾ Initiator:Campherchinon;Beschleuniger:N-(2-Cyanoethyl)-N-methylanilin;Inhibitor;Hydrochinonmonomethylether | | | | | |

**Tabelle 2**

| **Zusammensetzung der Kompositpasten** | | | | | |
|---|---|---|---|---|---|
| Bestandteil | Komposit (in Masse-%) | | | | |
| | 1 | 2^{*)} | 3 | 4^{*)} | 5^{*)} |
| Monomermischung₁₎ | 22,0 | 22,1 | 22,3 | 22,3 | 22,3 |
| Alkaliglas, sil.²⁾ | 48,0 | 52,2 | - | - | - |
| SP-2034, sil.³⁾ | 11,0 | - | 60,1 | 60,1 | 60,1 |
| YbF₃ ⁴⁾ | 12,0 | 10,0 | 11,6 | 11,6 | 11,6 |
| Aerosil-OX-50®, silanisiert⁵⁾ | 4,0 | 3,8 | - | - | - |
| Sphärosil® sil.⁶⁾ | - | - | 6,0 | 6,0 | 6,0 |
| HDK-2000⁷⁾ | 3,0 | 2,4 | - | - | - |
| Ba-Glas sil. (GM 27884)⁸⁾ | - | 9,5 | - | - | - |

| | | | | | |
|---|---|---|---|---|---|
| ¹⁾ es wurden die in Tabelle 1 aufgeführten Monomermischungen verwendet, Komposit 1 basiert auf Monomermischung 1, etc. | | | | | |
| ²⁾ silanisiertes alkalisches Glas mit 47,4 Gew.-% SiO_{2,} 39,8 Gew,-% CaO, 8,4 Gew.-% Na₂O, 7,6 Gew.-% F⁻ | | | | | |
| ³⁾ silanisiertes Glasionomer-Fluor-Calcium-Aluminium-Silikatglas, mittlere Korngröße 1,6 µm | | | | | |
| ⁴⁾ Ytterbiumfluorid (Fa. Rhone-Poulenc) | | | | | |
| ⁵⁾ silanisierte Pyrolysekieselsäure (Fa. Degussa) Primärpartikelgröße 40 nm, BET-Oberfläche 50 m²/g | | | | | |
| ⁶⁾ silanisiertes SiO₂-ZrO₂-Mischoxid (Fa. Tokoyama Soda), Sekundärkorngröße < 7 µm | | | | | |
| ⁷⁾ hochdisperse Fällungskieselsäure (Fa. Wacker) | | | | | |
| ⁸⁾ silanisiertes Bariumaluminimsilikatglaspulver (Fa. Schott), Anteil mit einer Korngröße < 7 µm:99 % | | | | | |

Aus den Kompositpasten wurden Prüfkörper entsprechend ISO-Norm 4049 (1988) geformt, durch Bestrahlen mit Licht einer Wellenlänge von 400-500 nm (2 x 3 Minuten) gehärtet und anschließend deren mechanische Eigenschaften bestimmt.

Zur Ermittlung des Fluoridfreisetzungsvermögens wurden ausgehärtete Prüfkörper (Durchmesser = 20 mm, H = 1,5 mm) in 30 ml Pufferlösung bei 37°c im Schüttler gelagert und nach bestimmten Zeitabständen die freigesetzte Fluoridmenge mit einer Fluorelektrode gemessen.

Die in Tabelle 3 zusammengefaßten Ergebnisse zeigen, daß das hydrophile Komposit 1, das keine aciden oder inonischen Monomerkomponenten enthält, innerhalb von 28 Tagen etwa zehnmal mehr Fluoridionen freisetzt als Compoglass®, einem handelsüblichen Kompomer auf Basis COOH-acider Monomere (Füllstoff: SP-2034 und YbF₃). Die Fluoridionenfreisetzung von Komposit 1 liegt damit in der gleichen Größenordnung wie die von Glasionomerzementen (vgl. z.B. Vivaglass®, Glasionomerzement auf Basis von Polyacrylsäure, Füllstoff: SP-2034 und YbF₃).

Komposit 1 weist jedoch deutlich bessere mechanische Eigenschaften und eine höhere Wasserbeständigkeit als das Kompomer und der Glasionomerzement auf. Selbst sechstägiges Lagern in Wasser und anschließendes 24-stündiges Kochen beeinträchtigen die mechanischen Eigenschaften kaum.

Ungehärteter Komposit 1 war unter feuchteten Bedingungen (90 % Luftfeuchtigkeit) über den Untersuchungszeitraum von 8 Wochen lagerstabil.

Wird in Komposit 1 das hydrophile GDMA durch das hydrophobe TEGDMA ersetzt (Komposit 2), wird eine deutlich geringere Fluoridfreisetzung gemessen, obwohl Komposit 2 anstelle des SP-2034 einen größeren Anteil an Alkaliglas enhielt, das eine deutlich höhere Fluoridfreisetung als SP-2034 zeigt.

Komposit 3 enthält als ionenfreisetzenden Füllstoff ausschließlich SP-2034. Seine Fluoridfreisetzung ist mit der von Compoglass® vergleichbar, jedoch ist Komposit 3 im Gegensatz zu Compoglass® im ungehärteten Zustand auch unter feuchten Bedingungen lagerstabil.

In Komposit 4 wurde GDMA durch TEGDMA ersetzt. Wie im Fall der Komposite 1 und 2 bewirkt dieser Austausch eine deutliche Verringerung der Fluoridfreisetzung.

In Komposit 5 wurde das hydrophile jedoch monofunktionelle HEMA anstelle von GDMA verwendet. Komposit 5 zeigt zwar eine deutlich höhere Fluoridfreisetzung als Komposit 4, allerdings sind die mechanischen Eigenschaften nach Wasseranlagerung unbefriedigend.

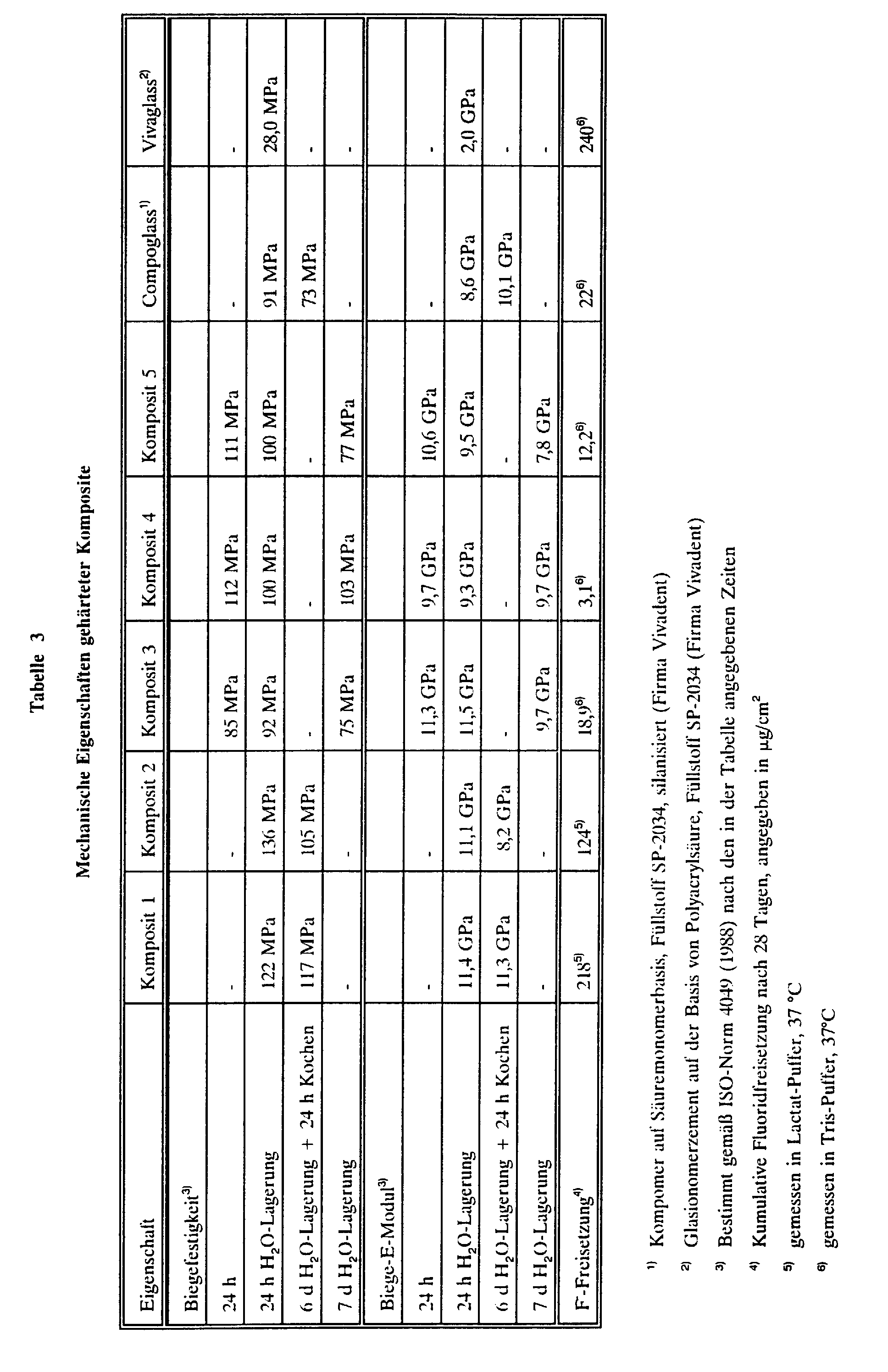

## Patentansprüche

1. Ionenfreisetzender Kompositwerkstoff auf Basis polymerisierbarer Monomere, **dadurch gekennzeichnet**, daß der Werkstoff eine Mischung aus
(a) mindestens einem nichtaciden, nichtionischen, hydrophilen Vernetzermonomer,
(b) mindestens einem nichtaciden, nichtionischen, hydrophilen Verdünnungsmonomer mit einer Viskosität < 1 Pas und
(c) mindestens einen Ionen freisetzenden Füllstoff enthält.

2. Kompositwerkstoff gemäß Anspruch 1, **dadurch gekennzeichnet**, daß er
(a) 1 bis 40 Gew.-% des oder der Vernetzermonomere;
(b) 2 bis 40 Gew.-% des oder der Verdünnungsmonomere;
(c) 30 bis 94 Gew.-% des Ionen feisetzenden Füllstoffs;
(d) 0,01 bis 5 Gew.-% eines radikalischen Initiators sowie ggf. weitere Hilfsstoffe enthält.

3. Kompositwerkstoff gemäß Anspruch 2, **dadurch gekennzeichnet**, daß er
(a) 10 bis 30 Gew.-% eines oder mehrerer Vernetzermonomere;
(b) 2 bis 30 Gew.-% eines oder mehrerer Verdünnungsmonomere; und/oder
(d) 0,1 bis 2,0 Gew.-% Initiator
enthält.

4. Kompositwerkstoff gemäß Anspruch 3, **dadurch gekennzeichnet**, daß er
(a) 15 bis 25 Gew.-% eines oder mehrerer Vernetzermonomere; und/oder
(b) 5 bis 20 Gew.-% eines oder mehrerer Verdünnungsmonomere enthält.

5. Kompositwerkstoff gemäß einem der Ansprüche 1 bis 4, **dadurch gekennzeichnet**, daß er 30 bis 60 Gew.-% (Zement) bzw. 60 bis 94 Gew.-% (Füllungskomposit) Füllstoff enthält.

6. Kompositwerkstoff gemäß einem der Ansprüche 1 bis 5, **dadurch gekennzeichent**, daß das oder die Vernetzer- und/oder Verdünnungsmonomere Urethan- und/oder OH-Gruppen enthalten.

7. Kompositwerkstoff gemäß Anspruch 6, **dadurch gekennzeichnet**, daß er als Vernetzermonomer 2,2-Bis-4-(3-methacryloxy-2-hydroxypropyl)-phenylpropan) (Bis-GMA), 7,7,9-Trimethyl-4,13-dioxo-3,14-dioxa-5,12-diazahexadecan-1,16-diyl-dimethacrylat (UDMA), ein Umsetzungsprodukt von Glycidylmethacrylat mit einem Bisphenol und/oder ein Umsetzungsprodukt von 2 mol 2-Hydroxyethylmethacrylat (HEMA) oder 2-Hydroxypropyl(meth)acrylat mit 1 mol Diisocyanat enthält.

8. Kompositwerkstoff gemäß einem der Ansprüche 1 bis 7, **dadurch gekennzeichnet**, daß er als Verdünnungsmonomer Glycerindimethacrylat (GDMA), ein Umsetzungprodunkt von niedrigviskosen Di- und Triepoxiden mit (Meth)acrylsäure und/oder ein Umsetzungsprodukt von 2 bzw. 3 mol Methacrylsäure mit Glycerintriglycidylether oder Trimethylolpropantriglycidether enthält.

9. Kompositwerkstoff gemäß einem der Ansprüche 1 bis 8, **dadurch gekennzeichnet**, daß das Vernetzungs- und/oder Verdünnungsmonomer Methacryl- und/oder Acrylgruppen als polymerisationsfähige Gruppen enthält.

10. Kompositwerkstoff gemäß einem der Ansprüche 1 bis 9, **dadurch gekennzeichnet**, daß er als Initiator Azobis(isobutyronitril) Azobis-(4-cyanvalerialnsäure), Dibenzoylperoxid, Dilauroylperoxid, tert.-Butylperoctoat, tert.-Butylperbenzoat, Di-(tert.-butyl)-peroxid, Benzpinakol, ein 2,2'-Di(C₁-C₈-alkyl)-benzpinakol, einen Benzoinether, ein Dialkylbenzilketal, Dialkoxyacetophenon, Acylphosphinoxid, 9,10-Phenanthrenchinon, Diacetyl, Furil, Anisil, 4,4'-Dichlorbenzil, 4,4'-Dialkoxybenzil und/oder Kampherchinon enthält.

11. Kompositwerkstoff gemäß einem der Ansprüche 1 bis 10, **dadurch gekennzeichnet**, daß er als ionenfreisetzenden Füllstoff einen Ca²⁺-, F⁻- und/oder OH⁻-Ionen freisetzenden Füllstoff enthält.

12. Kompositwerkstoff gemäß Anspruch 11, **dadurch gekennzeichnet**, daß er als Füllstoff ein Glaspulver eines Fluoraluminiumsilicatglases mit einer mittleren Partikelgröße von 0,05 bis 15 µm enthält.

13. Kompositwerkstoff gemäß einem der Ansprüche 1 bis 12, **dadurch gekennzeichnet**, daß er einen alkalischen Füllstoff enthält.

14. Kompositwerkstoff gemäß Anspruch 13, **dadurch gekennzeichnet**, daß er als Füllstoff Calciumhydroxid, Calciumoxid und/oder ein Calciumhydroxid freisetzendes Glaspulver enthält.

15. Kompositwerkstoff gemäß Anspruch 14, **dadurch gekennzeichnet**, daß er ein Glaspulver mit einem CaO-Gehalt von mindestens 20 Gew.-% enthält.

16. Kompositwerkstoff gemäß Anspruch 15, **dadurch gekennzeichnet**, daß das Glaspulver 24,0 bis 56,0 Gew.-% SiO₂, 26,0 bis 57,0 Gew.-% CaO und 4,0 bis 14,0 Gew.-% F enthält.

17. Verwendung eines Kompositwerkstoffs gemäß einem der Ansprüche 1 bis 16 als Dentalmaterial.
